(19) Europäisches Patentamt
European Patent Office
Office européen des brevets

(11) Publication number : **0 474 387 A1**

(12) **EUROPEAN PATENT APPLICATION**

(21) Application number : 91307600.6

(22) Date of filing : 16.08.91

(51) Int. Cl.⁵ : **C07C 49/17, C07C 45/75**

(30) Priority : **04.09.90 GB 9019311**

(43) Date of publication of application :
**11.03.92 Bulletin 92/11**

(84) Designated Contracting States :
**BE DE DK ES FR GB IT NL SE**

(71) Applicant : **BP Chemicals Limited**
**Belgrave House 76 Buckingham Palace Road**
**London, SW1W 0SU (GB)**

(72) Inventor : **Avison, Carl Anthony, BP Chemicals**
**Ltd.**
**Belgrave House, 76, Buckingham Palace Road**
**London, SW1W 0SU (GB)**
Inventor : **Dobson, Ian David**
**BP Chemicals Limited, Salt End**
**Hedon, Hull, HU12 8DS (GB)**
Inventor : **Gracey, Benjamin Patrick**
**BP Chemicals Limited, Salt End**
**Hedon, Hull, HU12 8DS (GB)**
Inventor : **Kikabhai, Thakor**
**BP Chemicals Limited, Salt End**
**Hedon, Hull, HU12 8DS (GB)**

(74) Representative : **Krishnan, Suryanarayana**
**Kalyana et al**
**BP INTERNATIONAL LIMITED Patents &**
**Agreements Division Chertsey Road**
**Sunbury-on-Thames Middlesex TW16 7LN**
**(GB)**

(54) **Synthesis of alpha-hydroxy ketones.**

(57)    This invention relates to a process for producing alpha-hydroxy ketones by condensation of one or more aldehydes in a liquid reaction system in the presence of an active condensation catalyst wherein the catalyst upon deactivation is reactivated by removal of water therefrom and recycled to the reaction system.
Dihydroxyacetone is a vital raw material for the production of glycerol.

EP 0 474 387 A1

This invention relates to a process for the production of alpha-hydroxy ketones, especially dihydroxy acetone and the use thereof for producing glycerol.

Dihydroxy acetone is a valuable raw material for the production of alcohols and esters, especially glycerol which in turn is used to produce various esters, printing inks, foodstuffs, in antifreezes, as a moistening agent in tobacco, in soaps and for producing nitroglycerine.

Our published EP-A-306215 describes a process for synthesising glycerol by initially self condensing formaldehyde in a substantially anhydrous state to dihydroxy acetone (hereafter DHA) followed by hydrogenation of the DHA. In this document, and in other prior published documents on self condensation reactions such as the article by Matsumoto, T. et al in J.A.C.S., 1984, 106, pp4289-4832 and in EP-A-245976, the self condensation stage has been carried out in the presence of a base such as triethyl amine and halide ions such as bromides. The bromide ions come from the salt of the heterocyclic catalyst such as the thiazolium bromide used. However, these processes are commercially unattractive because the catalyst used in such processes could not be readily recycled due to its deactivation by water, which can be a by-product of the process.

It is an object of the present invention to devise a simple route to alpha-hydroxy ketones e.g. DHA from aldehydes such as formaldehyde. A further object of the process is to provide a simple method of regenerating the deactivated condensation catalyst which can then be recycled to the reaction.

It has now been found that formaldehyde can be converted to DHA at significant rates and in good selectivities and yields to make an integrated process to glycerol and other commodity chemicals commercially attractive.

Accordingly the present invention is a process for producing alpha-hydroxy ketones by condensation of one or more aldehydes in a liquid reaction system in the presence of an active condensation catalyst characterised in that the catalyst upon deactivation is reactivated by removal of water therefrom and recycled to the reaction system.

According to another embodiment the present invention is a process for producing alpha-hydroxy ketones by condensation of one or more aldehydes in a liquid reaction system in the presence of an active condensation catalyst characterised in that the catalyst upon deactivation is recycled into the reaction system and then reactivated in situ by the removal of water therefrom.

By an "active condensation catalyst" is meant here and throughout the specification a catalyst system which includes an organic nitrogen containing species, having at least one carbon atom capable of inducing condensation of aldehydes so as to form alpha-hydroxy ketones as the major reaction product.

The active catalyst can be generated either before the commencement of the condensation reaction or during the condensation reaction.

The process of the present invention has only been made possible due to our surprising observation that the poisoning/deactivation of the active catalyst by water is a reversible reaction. Thus by removing water from the condensation reaction, the catalyst can be maintained in an active state. Furthermore a catalyst system which has been exposed to a considerable amount of water so as to render it inactive, can be regenerated by the removal of water from the catalyst system.

One further important aspect of the condensation reaction system is that certain side reactions which occur actually produce water. This means that without the ability to regenerate a catalyst system which has been exposed to water, the catalyst system will become inactive even if the reagents used were initially dry. The present invention therefore provides a method for reactivating in situ or for recycling and then regenerating catalyst deactivated by this route.

A further feature of the present invention is that the aldehyde reactant used can be hydrous or anhydrous prior to the commencement of condensation. However, during the actual condensation reaction, it is important to remove as much of the water from the reaction system as is practicable to achieve optimum results. Ideally, the reactants should be substantially dry, i.e. substantially free of water to maximise catalyst activity. One method of achieving such an objective is to use for the reaction e.g. a distillation still from which water is continually removed overhead whereas the reaction occurs at the bottom of the still, the so called "kettle" of the still. When using aqueous reactants, it is essential that there is present in the reaction system an organic solvent capable of (a) dissolving the formaldehyde reactant to provide a homogeneous liquid reaction medium and (b) removing overhead in the reactor any water present in the reactants or the catalyst as an azeotrope. Such will ensure that the reactants are substantially dry during the actual condensation reaction. Examples of azeotroping agents that can be used to aid water removal include alcohols, ethers or hydrocarbons. Such a process has the particular advantage that the spent or deactivated catalyst can be recycled as such and then reactivated in situ thus providing an unexpected and surprising advantage in that the catalyst need not be activated externally prior to reuse in the condensation reaction. Alternatively, water can be removed from the reaction system by other methods such as adsorption onto zeolites or alumina, membrane separation processes such as pervaporation, chemical drying reagents and others known to those skilled in the art of water removal.

A significant advantage of the present process where the catalyst is first regenerated and then recycled is that water can be removed continuously from the system in this manner. Thus, the continuous removal of water from the system has the added benefit of regenerating the catalyst thereby enabling recycle of the regenerated catalyst to the condensation stage. It may be necessary in some cases to supplement any such recycled catalyst with one or more of the essential components in order to maintain a uniform catalyst activity in the reaction.

Thus, if the aldehyde used is formaldehyde, it may be used in monomeric, oligomeric or polymeric form. In monomeric form it may be used either as formaldehyde gas or as a solution of formaldehyde in water or an organic solvent, suitably an alkanol, for example methanol, ethanol or propanol, butanol, cyclohexanol, methylisobutyl carbinol, 2-ethyl hexanol, glycols, such as ethylene glycol; polyols such as glycerol; glycol ethers such as methoxy propan-2-ol and glymes; amides such as dimethyl formamide; nitrogeneous solvents such as N-methyl pyrrolidone; esters such as gamma-butytrolactone; and hydrocarbons such as toluene or decane; or mixtures of such solvents.

The source of the active catalyst is suitably a thiazolium salt of counter ion X. It is preferably an aliphatic, aromatic or a heterocyclic thiazolium salt of the counter ion X. The counter ion X may be any ion capable of forming a salt with the thiazolium cation. Specific examples of such counter ion X include the halides, the sulphates and the carboxylates but is preferably a halide and is especially a bromide. Of these the 3-methyl benzothiazolium bromide, 3-ethylbenzothiazolium bromide, 3-laurylbenzothiazolium bromide, 3-isopropylbenzothiazolium bromide, 3-butylbenzothiazolium bromide and 3-ethyl thiazolium bromide are specific examples. The thiazolium ion is chosen so as to offer an optimum combination of catalyst activity and ease of separation from the desired hydroxyacetone product.

Active condensation catalysts can be prepared from the above sources in a variety of ways.

Most commonly an active catalyst is prepared from a nitrogen containing heterocyclic quaternary ammonium salt such as the thiazolium salt, and a base capable of abstracting a proton from the quaternary ammonium salt, such that the moiety $H^+X^-$ is removed from the quaternary ammonium salt where X is the counter ion. Typical of this approach is the generation of an active catalyst from an N-alkylbenzothiazolium halide plus a simple amine base such as triethylamine.

More advantageously, a basic ion exchange resin can be used instead of a simple amine. In this case an active condensation catalyst can be prepared with the added benefit of being-readily separated from halide ions and the basic ion exchange resin before use.

Another method is to thermally decompose a suitable catalyst precursor. For example 4,5-dimethyl-N-ethylthiazolium-2-carboxylate is readily decarboxylated by heating to give very cleanly an active condensation catalyst.

A further approach is to add a reagent to remove a group other than HX from a catalyst precursor. An example of this method would be the use of fluoride ions to remove the trimethylsilyl group from a catalyst precursor such as the 2-trimethylsilyl-3-alkylthiazolium ion.

Furthermore, active catalyst systems in which more than one heterocyclic ring structuure is present can be produced. For example 1,3-diarylimidazolinium salts and base can be used to generate this type of active condensation catalyst. Similarly, bis-benzothiazolium based catalysts can also be prepared.

The relative molar ratios of the reactant aldehyde to the condensation catalyst in the initial reaction mixture may vary over a wide range. Use of excess catalyst is not detrimental to the reaction or the economics of the process since the catalyst can be regenerated and reused. Thus the mole ratio of the aldehyde reactant to catalyst can be as low as 3:1 or as high as 10000:1.

The liquid medium used in the reaction system is preferably a (cyclo) aliphatic alcohol. The (cyclo) aliphatic alcohol solvent for the condensation step is suitably a solvent or mixtures of solvents capable of dissolving the reactant aldehyde and/or the active condensation catalyst in the system. In a homogeneous system clearly the solvent will dissolve both.

The liquid medium used in the reaction is suitably a solvent or mixture of solvents capable of wholly or partially dissolving the reactant aldehyde and/or the active condensation catalyst in the system. In a homogeneous system clearly the solvent will dissolve both. The preferred liquid medium is an alcohol or mixture of alcohols. Suitable alcohols include for example, methanol, ethanol, n-propanol, iso-propanol, n-butanol, iso-butanol, sec-butanol, cyclohexanol, methylisobutylcarbinol and 2-ethylhexanol including those listed above as solvents for formaldehyde. The particular choice of liquid medium will depend on the chosen method of separating the reaction product from the catalyst system which is to be recycled. Use of cycloaliphatic alcohols is preferred.

The condensation reaction can be a self-condensation or cross-condensation. In the case of the latter at least two aldehyde reactants should be present. The self-condensation is preferably carried out with formaldehyde and in this case the product is dihydroxy acetone ("DHA"). The self-condensation of formaldehyde is suitably carried out at a temperature from 20-200°C, preferably from 80-170°C, most preferably from 100-120°C. It will be appreciated that the reaction is exothermic and hence reactions initiated within this range may, during the reaction, exceed the preferred upper limit of 170°C specified herein. The reaction pressure may be reduced, ambient or elevated provided that the pressure is controlled to maintain the reactants and/or solvents substantially in the liquid state. Variation of reaction pressure may be usefully employed to control the temperature in the reaction zone and hence to control the reaction rate.

The self-condensation product containing dihydroxy acetone as such, as a dimer thereof or as a mixture of the two can be separated from the catalyst components by conventional means e.g. by one or more of the following: precipitation, dialysis, liquid-liquid extraction, membrane separation such as hyperfiltration, distillation (whether or not under vacuum) e.g. steam stripping, and the use of adsorbent materials such as ion exchange resins.

In the case of certain solvents, e.g. alcoholic solvents such as 2-ethyl hexanol or methylisobutyl carbinol, used in the self-condensation stage, the dihydroxy acetone formed can be separated from the reaction mixture simply by cooling whereby the dihydroxy acetone precipitates. The remaining liquid phase can be optionally recycled to the condensation reactor or further processed in the separation stage. Precipitation can be made to occur so as to recover a greater proportion of the dihydroxyacetone or its dimer than is achieved simply by cooling. One method is to reduce the volume of the solvent present by distillation and then cool the remaining solution. An alternative approach is to remove essentially all of the solvent to give a solid or a viscous liquid phase. This phase is then redissolved in a solvent from which dihydroxyacetone or its dimer can be readily precipitated either by cooling or by the addition of a second liquid component such as e.g. additions of diethyl ether or 2-ethylhexanol to a solution of the viscous phase in acetone. For this method a volatile alcohol such as n-propanol is particularly suitable as the reaction medium.

Under certain temperature or dihydroxyacetone concentration regimes it is possible to substantially separate the dihydroxyacetone from the reaction solvent as a liquid phase precipitation. A dihydroxyacetone rich liquid phase can thus be removed for further processing leaving a solvent rich phase for recycle to the condensation stage.

If it is intended to use liquid-liquid extraction as the separation method, then water can be used as the extractant to preferentially extract dihydroxyacetone from reaction solvents such as 2-ethyl hexanol. In this case, the efficiency of separation of dihydroxyacetone can be improved by using as catalyst a thiazolium salt bearing large aliphatic groups thereby enhancing the partition of the catalyst into the organic phase, from which the water deactivated catalyst is readily recycled to the condensation reaction kettle for regeneration.

Adsorbent materials may also be used to remove the catalyst system from the reaction products either alone or in combination with any one of the above methods. Typical adsorbents include activated carbons, alumina, silica, metal oxides, supported metals on carbon or metal oxide and ion exchange resins. With certain adsorbent materials, it can be advantageous to regenerate the adsorbent, for example by heating the adsorbent in a suitable atmosphere such as hydrogen, steam or air. Adsorbent treatments are particularly suitable for use in combination with a treatment such as liquid-liquid extraction.

The DHA so recovered is suitable for use particularly as a source of glycerol upon hydrogenation.

It is a feature of the invention that, depending upon the method of recovery of DHA, the sulphur, nitrogen and halogen containing components can be substantially absent from the reaction products of the condensation step, and hence the remaining products containing crude dihydroxy acetone need not be further purified prior to use in the hydrogenation step for converting DHA to glycerol. One such method where the crude DHA product can be used directly for hydrogenation to glycerol is that recovered by crystallisation.

As regards the hydrogenation step, the dihydroxyacetone can be hydrogenated in the presence of a hydrogenation catalyst and hydrogen. The hydrogenation catalyst may be a heterogeneous or a homogeneous hydrogenation catalyst.

Where the catalyst is a heterogeneous hydrogenation catalyst it is suitably a finely divided or a supported Group VIII metal. For example such a catalyst may be nickel, Raney nickel or ruthenium supported on a support inert under the reaction conditions e.g. carbon or graphite, or copper chromite type catalyst. Where the hydrogenation catalyst is a homogeneous catalyst, such a catalyst is soluble in the liquid reaction medium and is suitably a compound or mixture of compounds containing a noble metal moiety (i) and a moiety (ii) of the formula $QR_3$ wherein Q is either phosphorus, arsenic or antimony and the groups R are independently either hydrogen or hydrocarbyl or substituted hydrocarbyl groups. Throughout this specification the term noble metal means platinum, palladium, rhodium, ruthenium, iridium or osmium. Of the noble metals, palladium, platinum, rhodium and ruthenium are preferred. Preferably Q in the formula is phosphorus. The group R in the formula is preferably a hydrocarbyl or substituted hydrocarbyl group. Suitable hydrocarbyl groups include alkyl groups, cycloalkyl groups and aryl groups, which may be substituted or unsubstituted. The catalyst may suitably combine the moieties (i) and (ii) in a single compound, for example as the compound $RhCl(PPh_3)_3$ or the compound $Ru(H)(OAc)(PPh_3)_3$. Alternatively, the moities (i) and (ii) may be added in the form of separate compounds for example as $RhCl_2$ and $PPh_3$ to form the hydrogenation catalyst in situ.

Where the catalyst is a homogenous hydrogenation catalyst, particularly when this takes the form of a single compound, it may be supported on a suitable support. Such supports include organic polymers, for example polystyrene containing the appropriate functional moiety (ii).

Hydrogen is readily available on a commercial scale. It may be used in a commercially available form or may, if desired, be further purified. The hydrogen partial pressure is suitably in the range from 10 to 30,000 KPa, preferably from 100 to 5000 KPa.

The hydrogenation of DHA may be accomplished at elevated temperature, suitably in the range from ambient to 150°C, preferably from 40 to 150°C, most preferably from 40 to 120°C.

The liquid reaction medium for the hydrogenation step is suitably a solvent capable of dissolving the hydrogenation reactants and, in the case of a homogeneous reaction, the catalyst. Suitable solvents include, but are not restricted to, alcohols, water, ethers and mixtures of one or more of these. The particular solvent of preference may be advantageously the same as that chosen for the self condensation step.

The hydrogenation of DHA may suitably be carried out batchwise or continuously, preferably continuously.

For a batch operation, the duration of the hydrogenation reaction will vary with the type and concentration of the hydrogenation catalyst, the hydrogen partial pressure and with the nature of the product being hydrogenated, i.e. whether crude or pure or whether the reaction is carried out in situ. The glycerol product formed upon hydrogenation can be purified and recovered by methods well known in the art. A suitable method of purification is vacuum distillation. If required post treatments known to those skilled in the art may be used. Such treatments include, but are not restricted to, passage over a carbon bed and treatment with bleaching agent.

The present process is clearly simpler and less expensive to operate than the synthetic processes used hitherto. The raw materials are readily available and the products easily separated and purified.

The present invention is further illustrated with reference to the following Examples.

Example 1

This Example demonstrates the recycling and reactivation of a deactivated condensation catalyst in which the catalyst is deliberately brought into contact with water during the liquid extraction step to remove the dihydroxyacetone (DHA) product.

A product stream was generated by passing a reactant stream comprising 3-ethylbenzothiazolium bromide (0.6mmol per 100g solvent) and 26wt% formaldehyde (HCHO) in 2-ethylhexanol (2EH), through a bed of Amberlyst A21 weakly basic ion exchange resin (10g dry weight) at a range of temperatures between 30-90°C using a flow rate of 35-40ml/hr and heating the mixture thereafter (30 mins at 117°C). In addition, following treatment with ion exchange bed, the level of bromide is reduced from 504 ppm (w/w) to 4 ppm (w/w) demonstrating the ability of the resin to remove bromide from the reaction stream. The resin bed was pretreated by drying 15g of Amberlyst A21 in vacuo, pre-swelling overnight in the HCHO/2EH solution and washing with 2EH prior to the experiment. The pretreated resin was packed into a glass column and between 10ml sections of glass beads. The column was chosen such that the length:diameter ratio was >10.

A product stream sample was prepared using a resin bed temperature of 70°C. The product stream comprised 1.85wt% dihydroxyacetone (DHA) and 0.2wt% erythrulose hydrate. The product stream was extracted with water to remove DHA and erythrulose hydrate (2 successive extractions; ratio product stream:water = 5:1v/v, 1 min contact time, manual shaking, 1 day settling time). The extract was then allowed to separate into aqueous and organic layers, the organic layer containing the catalyst which would have been deactivated by the presence of the extractant water (used for the recovery of DHA) partitioning into the organic layer. The organic layer (215.3g) containing the recycled deactivated catalyst was transferred to a 500ml flask, purged with nitrogen and heated (106-7°C) such that an organic/water azeotrope was continuously removed from the system. The reaction was regularly sampled by withdrawal by syringe through a septum fitted to the reaction flask. The following results were obtained.

| TIME (MINUTES) | FORMALDEHYDE (%W/W) | DIHYDROXYACETONE (% W/W) |
|---|---|---|
| 0 | 21.6 | <0.1 |
| 15 | 21.39 | <0.1 |
| 30 | 20.97 | <0.1 |
| 60 | 20.6 | <0.1 |
| 120 | 19.09 | <0.1 |
| 180 a) | 11.17 | 1.25 |
| 270 b) | 6.49 | 6.03 |
| 300 b) | 5.65 | 9.30 |
| 370 b) | 4.6 | 11.02 |
| 420 b) | 4.20 | 11.95 |

a) Solution was pale yellow.

b) Solution was yellow, white precipitate (DHA) appeared on cooling.

It can be seen that immediately following recycle the reaction system was not catalytically active; activity however returned between 120 and 180 minutes into the reaction procedure thus confirming that the activation of the recycled deactivated catalyst had taken place in situ.

## Example 2

A reaction mixture was prepared by dissolving 3-ethylbenzothiazolium bromide (6mmol per 100g solvent), triethylamine (5.4mmol per 100g solvent) and 21.7wt% HCHO (7.5wt% water) in 4-methyl pentan-2-ol(MIBC) using a magnetic follower. This reaction mixture (768.4g) was added to a 1000ml flask, purged with nitrogen and heated (110°C) such that an organic/water azeotrope was continuously removed from the system. After 3 hours, the mixture was sampled and analysed. The mixture contains 4.6wt% DHA and 0.04wt% HCHO. The solution was allowed to cool overnight, whereupon some DHA precipitated out of solution.

The supernatant liquid (263.6g), paraformaldehyde (26g) and water (6g) were transferred to a 500ml flask and heated to 80°C for 3 hours to allow HCHO to form the alcohol hemiformal (clear solution). This mixture was analysed and found to contain 3.7wt% DHA. The flask was then purged with nitrogen and heated to 110°C such that an organic/water azeotrope was continuously removed from the system. After 1 hour the solution was sampled and analysed. This mixture contained 4.0wt% DHA demonstrating that further condensation of HCHO had occurred.

The above example demonstrates the use of the invention to reactivate a condensation catalyst following the separation of dihydroxyacetone by precipitation/filtration and the addition of further formaldehyde.

## Example 3

This example outlines the continuous synthesis and extraction of DHA on the pilot plant.

## Description of the Pilot Plant Unit

Essentially, the unit comprises of a reactive distillation column and a liquid extraction column.

The reactive distillation column was a 35 glass Oldershaw plate, vacuum jacketed glass tower with an internal diameter of 80 mm. The tower was constructed from 3 10-plate and 1 5-plate sections. A 316 ss reactor (also known as the reboiler, kettle or still) was fitted to the bottom of the column. The reboiler was of the thermosyphon type with two heating legs connected to a central main body. Power input was supplied via both external windings and internal cartridge heaters for each leg of the reboiler. Level indication was supplied via an integral sight glass arrangement together with an external level transmitter connected to an automatic pump. Nominally, the reboiler had a capacity of 2.5 litres.

The liquid extraction tower was a 10 m (100 mm internal diameter) glass column packed with ceramic raschid rings. Due to physical limitations, the tower was separated into two identical 5 m extractors interconnected by a buffer tank and pumps.

## Description of the Process

Formaldehyde was extracted from commercial aqueous formalin stabilised with isobutanol (30 wt% HCHO, 6 wt% $^i$BuOH) into a mixed isobutanol/methylisobutylcarbinol, MIBC (50:50 v/v) stream in the liquid-liquid extractor column. The formalin was added to the top of the liquid extractor at a flow rate of 1 litre/hr at ambient temperature. The formaldehyde rich organic stream (typically comprising 20 wt% HCHO, 32 wt% $^i$BuOH, 41 wt% MIBC), from the top of the extractor, was added to plate 20 of the distillation column at a rate of ca 2.2 litre/hr.

The active catalyst was prepared separately, prior to the condensation reaction, by passing a concentrated solution of 3-ethyl benzothiazolium bromide through a 1 m (50 mm diameter) glass column packed with Amberlyst A21 (Registered Trade Mark, a commercial ion exchange resin). This procedure activated the catalyst and removed bromide from the stream. Typically, the ion exchanged solution was analysed and found to contain 2803 ppm (w/w) S and < 10 ppm (w/w) Br. The solution was further concentrated by rotary evaporation at 40°C and slightly reduced pressure to remove excess isobutanol. Typically, this final concentrated solution comprised 6600 ppm (w/w) N.

A concentrated solution of active catalyst (3-ethylbenzothiazolium zwitterion) in isobutanol was added to the distillation column (at plate 10) prior to the reaction conditions being reached such that the kettle (at the bottom of the distillation column) comprised 2.3 wt% of the active catalyst as the reaction commenced.

Once the active concentrated catalyst was added to the condensation reaction system (the organic stream containing formaldehyde was continuously being added from the top of the extractor to the distillation column at a rate of 2.2 litre/hr), the kettle temperature was raised (with control being maintained by varying the pressure) to 118°C and held constant (+/- 2°C) throughout the experiment. During this experiment, a pressure of -0.2 bar absolute was required to maintain the kettle temperature between 118°C and 120°C.

Under the reaction conditions, the isobutanol continuously azeotroped the water (present in the feed streams and formed during the reaction stage) away from the reaction zone (ie the kettle). Typically, the water content of the kettle mixture was 0.02 wt% (determined by the Karl Fischer analytical method) during the experiment. The substantial absence of water ensured that the catalyst remained in its active form and condensed the formaldehyde to DHA in the kettle. This product was pumped from the reboiler to the mid point of the liquid-liquid extractor at a rate of ca 2.2 litre/hr. The isobutanol/water azeotrope was collected from the top of the column into a decanter tank and a reflux tank. From the decanter, an aqueous rich phase was pumped (at a rate of ca 0.34 litre/hr) to the liquid extractor to a point 3/4 from the base of the extractor. The organic rich phase, from the refux tank, was returned to the top of the distillation column at a rate of ca 0.68 litre/hr. These flows gave a reflux ratio of 2:1 for the distillation column.

Hence, during the process, the organic stream was pumped from the kettle to the liquid-liquid extractor column where products (such as DHA and erythrulose) were extracted into water and further formaldehyde was extracted into the organic stream. The catalyst preferentially remained in the organic stream in this process. The organic stream, comprising formaldehyde and catalyst, was then recycled to the distillation column.

## Experimental Results

The experiment lasted over 100 hours with the contents of the kettle being periodically monitored by high pressure liquid chromatography (HPLC). This analytical method identified DHA (between 5.3 wt% and 2.3 wt%) and the main reaction by-product, erythrulose (between 0.9 wt% and 0.1 wt%) throughout the course of the experiment. This corresponded to an internal selectivity of between 83% and 97% for the reaction. The analyses are shown below:

| Reaction Time (Hours) | DHA (wt%) | Erythrulose (wt%) | Catalyst (wt%) | MIBC (wt%) | $^i$BuOH (wt%) | Water (wt%) | DHA Selectivity (%) |
|---|---|---|---|---|---|---|---|
| 0 | 2.28 | 0.08 | 2.17 | NA | NA | 2.86 | 96.7 |
| 4 | 2.35 | 0.08 | 2.07 | NA | NA | 2.07 | 96.8 |
| 52 | 3.72 | 0.22 | 0.88 | 63.5 | 24.9 | 0.02 | 94.7 |
| 57 | NA | NA | 0.77 | 62.32 | 25.0 | 0.15 | NA |
| 58 | 6.39 | 0.16 | 0.77 | 65.24 | 25.85 | 0.02 | 97.7 |
| 92 | 5.29 | 0.37 | 0.74 | 52.79 | 30.14 | 0.04 | 93.8 |
| 108 | 5.02 | 0.26 | 0.72 | 49.56 | 31.71 | 0.02 | 95.3 |
| 113 | NA | NA | 0.72 | 49.19 | 30.85 | 0.02 | NA |
| 124 | 4.97 | 0.18 | 0.71 | 53.90 | 31.38 | 0.04 | 96.6 |
| 128 | 4.25 | 0.12 | 0.70 | 54.24 | 30.44 | 0.01 | 97.3 |

NA      = Not measured

MIBC   = Methyl isobutyl carbinol

$^i$BuOH = iso-butanol

## Claims

1. A process for producing alpha-hydroxy ketones by condensation of one or more aldehydes in a liquid reaction system in the presence of an active condensation catalyst characterised in that the catalyst upon deactivation is reactivated by removal of water therefrom and recycled to the reaction system.

2. A process for producing alpha-hydroxy ketones by condensation of one or more aldehydes in a liquid reaction system in the presence of an active condensation catalyst characterised in that the catalyst upon deactivation is recycled into the reaction system and then reactivated in situ by the removal of water therefrom.

3. A process according to Claim 1 or 2, wherein the active condensation catalyst is generated either before or during the condensation reaction.

4. A process according to any one of the preceding Claims wherein the condensation reaction is carried out in a distillation still from which water is continually removed overhead in order to achieve regeneration of the catalyst in situ.

5. A process according to Claim 4 wherein the water is removed overhead from the still as an azeotrope in the presence of an azeotroping agent.

6. A process according to Claim 5 wherein the azeotroping agent is selected from alcohols, ethers and hydrocarbons.

7. A process according to Claim 1 or 2 wherein the water is removed from the reaction system by a process selected from adsorption onto a zeolite or alumina, a membrane separation process and using chemical drying agents.

8. A process according to any one of the preceding Claims wherein the aldehyde being condensed is formaldehyde.

9. A process according to any one of the preceding Claims wherein the active catalyst system is derived from a heterocyclic nitrogen containing quaternary ammonium salt of counter ion X by abstraction of $H^+X^-$ therefrom.

10. A process according to any one of the preceding Claims 1-8 wherein the relative molar ratios of the reactant aldehyde to the condensation catalyst is from 3:1 to 10000:1 in the initial reaction mixture.

11. A process according to any one of the preceding Claims wherein the liquid medium used is a (cyclo) aliphatic alcohol capable of wholly or partially dissolving the reactant aldehyde and/or the active condensation catalyst in the reaction system.

12. A process according to any one of the preceding Claims wherein the reaction is a self-condensation reaction which is carried out at a temperature from 20-200°C.

13. A process according to Claim 13 wherein the dihydroxyacetone is produced by the self-condensation of formaldehyde.

**European Patent Office**

## EUROPEAN SEARCH REPORT

Application Number

| | DOCUMENTS CONSIDERED TO BE RELEVANT | | EP 91307600.6 |
|---|---|---|---|

| Category | Citation of document with indication, where appropriate, of relevant passages | Relevant to claim | CLASSIFICATION OF THE APPLICATION (Int. Cl.5) |
|---|---|---|---|
| P,X | <u>EP - A1 - 0 410 613</u> (BP CHEMICALS) * Claims 1,5-12,14-16 * | 1,2,5, 7,9-13 | C 07 C 49/17 C 07 C 45/75 |
| D,A | <u>EP - A2 - 0 306 215</u> (BP CHEMICALS) * Claims 1,4,5,15 * | 1,12, 13 | |
| D,A | <u>EP - A1 - 0 219 317</u> (THE BRITISH PETROLEUM) * Claims 1,10 * | 1 | |
| D,A | <u>EP - A1 - 0 245 976</u> (IMPERIAL CHEMICAL INDUSTRIES) * Claims 1,13 * | 1 | |

TECHNICAL FIELDS SEARCHED (Int. Cl.5)

C 07 C 45/00
C 07 C 49/00
C 07 C 29/00
C 07 C 31/00

The present search report has been drawn up for all claims

| Place of search | Date of completion of the search | Examiner |
|---|---|---|
| VIENNA | 12-11-1991 | REIF |